# EUROPEAN PATENT SPECIFICATION

(11) **EP 1 776 382 B1**
(45) Date of publication and mention of the grant of the patent: **03.12.2008**
(21) Application number: 05761444.8
(22) Date of filing: 20.07.2005
(51) Int. Cl.: C07K 14/47, C07K 1/107

(54) **A PROCESS FOR PRODUCING IRON SUCCINYL CASEIN AND ACETYL-ASPARTATE IRON CASEIN COMPLEXES AND USE THEREOF IN PHARMACEUTICAL MIXTURES**
VERFAHREN ZUR HERSTELLUNG VON EISENSUCCINYLCASEIN UND ACETYLASPARTAT-EISEN-CASEIN-KOMPLEXEN UND DEREN ANWENDUNG IN PHARMAZEUTISCHEN MISCHUNGEN
PROCEDE DE PRODUCTION DE COMPLEXES DE CASEINE SUCCINIQUE DE FER ET DE CASEINE DE FER D'ASPARTATE D'ACETYLE ET LEUR UTILISATION DANS DES MELANGES PHARMACEUTIQUES

(30) Priority: 29.07.2004 IT MO20040200
(43) Date of publication of application: 25.04.2007
(73) Proprietor: Biofer S.p.A., 41036 Medolla (IT)
(72) Inventor: LAPINI SACCHETTI Alessandro, I-41100 Modena (IT)
(74) Representative: Bottero, Carlo
(86) International application number: PCT/IB2005/002199
(87) International publication number: WO 2006/021843

(56) References cited:
- EP-A- 0 243 322
- US-A- 4 493 829
- CREMONESI P ET AL: "Iron derivatives of modified milk protein." ARZNEIMITTEL-FORSCHUNG. 1984, vol. 34, no. 9, 1984, pages 948-952, XP001207730 ISSN: 0004-4172
- CREMONESI P ET AL: "CHEMICAL AND BIOLOGICAL CHARACTERIZATION OF IRON-PROTEIN SUCCINYLATE (ITF 282)" INTERNATIONAL JOURNAL OF CLINICAL PHARMACOLOGY, THERAPY AND TOXICOLOGY, DUSTRI- VERLAG FEISTLE, DEISENHOFEN/MUENCHEN, DE, vol. 31, no. 1, January 1993 (1993-01), pages 40-51, XP001012289 ISSN: 0174-4879

## Description

### Technical Field

The aim of the invention is to provide an industrial process for production of iron succinyl casein and acetyl-aspartate iron casein based complexes in which the substance is little exposed to pH and temperature conditions which might damage the protein's secondary, tertiary and quaternary chemical-physical characteristics, as well as facilitating preparation of the relative liquid pharmaceutical formulations and their appearance as clear solutions.

The process is also easy to realise industrially and is simpler with respect to other known processes.

### Background Art

Iron plays a fundamental role in both human and animal physiology.

Its role and the lack of it in some pathological conditions make it important to administer iron-based substances in order to complete or replace quantities thereof normally assumed in the course of a normal diet.

Unfortunately many substances used in medical practice include relevant problems related to side effects.

Two pharmaceutical substances which have demonstrated fewer side effects are iron succinyl casein and acetyl-aspartate iron casein. Their success in some countries demonstrates the favourable reaction of the medical profession as well as of the patients.

In European patent application no. 98124195.3 (Bonifacio, Massardo, Di Leo), an improved process is described for the production of iron succinyl casein with respect to the process described in patent no. GB-A-2115821. However the process includes the use of machines and process conditions which are very specific and which limit productivity too, as they require realisation times which are very long, as well as continuous checks to be sure that temperature, pH and defined residual pressure parameters are all adhered to.

Further, by exposing the product for long times to non-optimal pH and temperatures for maintaining its protein structure, it is easy to damage the quality of the pharmaceutical substance with, for example, the formation of insoluble residue, as evidenced in European patent no. 98124195.3.

Also, Italian patent no. 1207996, relating to the production of acetyl-aspartate iron casein exhibits the same drawbacks as above for the iron succinyl casein complex.

The process described in the present patent application is differentiated from the prior art by the fact of the elimination of the drying stage of the final product, with machines requiring exposure of the product for prolonged periods to high temperatures, which thus obtains the product directly in the form of water-soluble sodium salt, which is the salt used in the final pharmaceutical mixture, thus avoiding the need to re-dissolve the product by addition of caustic soda, which adds to the danger of damaging it. Solublising apparatus is also required to guarantee an optimal and constant pH for the solution destined to be used to fill phials; the present invention does not require this apparatus. Also, no use of special pumps is required - pumps which may not always be readily available in the chemical industry and requiring high levels of maintenance.

### Disclosure of Invention

The invention relates to a process for obtaining iron succinyl casein and acetyl-aspartate iron casein complexes, obtained by reaction of high-quality casein, for pharmaceutical use, with succinyl anhydride and iron chloride or with acetyl aspartic anhydride and iron chloride.

The process described for obtaining iron complexes with succinyl casein and acetyl-aspartate casein is characterised in that:
a) the succinyl casein, iron succinyl casein, acetyl-aspartate casein and iron acetyl-aspartate casein obtained are damp-granulated when they are destined for further dissolving, to facilitate the dissolving process in water with addition of caustic soda with a pH of below eleven.
b) The purified iron succinyl casein and acetyl-aspartate iron casein complexes obtained are dried using a spray drier.

The spray-drier is a frequently used machine in the food industry, especially in milk-derivative products which include casein.

The spray-drier technique is used in the pharmaceutical industry whenever a heat-sensitive product is to be dried.

In spray-drying, the solution containing the iron casein complexes is reduced to very small droplets which are then dried by means of hot air which is directed onto the small droplets.

The powders thus obtained are collected in a separator centrifuge and then in a suitable container.

Alternatively, the solution containing the iron casein complexes can be freeze-dried. Freeze drying is obviously very gentle in terms of exposing the product to high temperatures. It is, however, an expensive process and is in general used for high-value products, almost always destined for use in the form of injections.

Overall the process of synthesis of the iron succinyl casein and iron acetyl-aspartate casein complex comprises the following steps:
1) The casein of suitable purity for a pharmaceutical process, dissolved with water with an addition of caustic soda having a pH of less than eleven, preferably less than 9, is made to react with succinyl anhydride or acetyl-aspartate anhydride in order to obtain succinyl casein or acetyl-aspartate casein which are precipitated for acidification at a pH of between 2.5 and 3.
2) The succinyl casein and acetyl-aspartate casein obtained in step 1 are dissolved in water with addition of caustic soda with a pH of less than 11, preferably less than 9, and are then reacted with iron chloride to obtain the iron succinyl casein and iron acetyl-aspartate casein complexes which precipitate from the solution when a pH of 2.5 to 3 is reached.
3) The complexes obtained in step 2 can be purified by pulping in water or alternatively by dissolving in water and caustic soda at a pH of less than 11, followed by a further precipitation at pH 2.5 to 3. The iron succinyl casein or iron acetyl-aspartate casein complexes are then dissolved in water with the help of caustic soda at a pH of less than 11, preferably less than 9, and are then added to by suitable preservatives (e.g. parabens) before being sent on to a spray-drier, where a powder is obtained that is directly usable for the pharmaceutical mixtures it is destined for.

Alternatively the solution can be sent on to a freeze-drying process.

The product has an iron complex content of between 4.8 and 5.5%, calculated by weight of dry substance. For the complete characterisation of the product, see the data included in examples nos. 1 and 2.

The described process, apart from being simpler than those described in the prior art, enables a product which is especially free of poorly-water-solvent parts, mainly due to denaturing of the protein structure.

Example no. 1. Production of iron succinyl casein complex.

### Step a) Succinylation of casein

In a stainless steel reactor with special stirrer (preferably an anchor stirrer) and pH detector, 300 litres of de-ionized, microbiologically pure water added to by 22 Kg of casein suitable for food or pharmaceutical use are mixed and shaken to obtain a homogeneous suspension. 2.2 litres of caustic soda at 15% w/v are added to this over a period of about 20-30 minutes up until a pH of about 8 is reached. To this solution are added 6.6 Kg of succinyl anhydride and about 16 litres of a 15% caustic soda solution, maintaining pH of between 7.5 and 9.

The solution obtained is left to rest for between 1 and 2 hours. The reaction is conducted at a temperature of between 20 and 25 degrees celsius.

### Step b) Precipitation of the succinyl casein

The solution obtained in step a) is transferred into a glaze reactor, equipped with a high-speed shaker to keep the solution homogeneous. This solution is slowly added to with 16-17 litres of HCl at 15% w/v up until a pH of about 3 is obtained. On reaching the correct pH addition of acid is stopped and a precipitate without salt and water-soluble impurities is obtained.

### Step c) Filtration and granulation.

The succinyl casein obtained in step b) is collected by filtration and washed with distilled water.

The product gathered from the filter is generally conformed in compact blocks which dissolve with difficulty.

To facilitate dissolving damp granulation (e.g. in a Viani granulator) of the filtered product is recommended.

The granulate is placed in a stainless steel reactor in which 300 litres of distilled water have been placed, and the mixture is shaken until a homogeneous suspension is obtained, to which about 7 litres of caustic soda at 15% w/v are added up until a pH of 8-9 is reached. During the final stage the suspension is shaken for about 6 hours in order to obtain an almost-complete dissolving of the succinyl casein.

The solution is sent on to filtration.

The filtered solution is loaded in a glaze reactor equipped with a fast shaker and with a pH detector, wherein over a period of about 30-40 minutes a solution of 6.4 ferrous chloride in 66 litres of water is added, up until a pH of 3 is reached.

When the pH of 3 is reached, addition of ferrous chloride is stopped. An iron succinyl casein complex precipitate is obtained, which is slowly shaken for about 30 minutes, whereupon the complex is filtered to gather the product thus obtained.

The reactor can be washed with distilled water to recuperate any product remaining in the reactor.

### Step d) Purification of the iron succinyl casein complex.

The iron succinyl casein complex obtained in step c) subjected to damp granulation is then loaded into a steel reactor where 350 litres of distilled water have been loaded.

The mixture is shaken for about 30 minutes to dissolve the salts and other water-soluble impurities and is then filtered.

### Step e) Clarification and spray-drying.

The washed and filtered and possibly granulated product is loaded into a steel shaker reactor into which 330 litres of distilled water have been loaded, and stirred for about an hour, whereupon 3.3 litres of a 15% caustic soda solution are added up until a pH of about 6 is reached.

The preservatives are added to this suspension, for example 1.46 kg of methylparaben in 5.5 litres of water and 0.4 kg of propylparaben in 5.5 litres of water.

About another 1 litre of 15% caustic soda solution is added to this suspension up until a pH of between 8 and 9 is reached.

During the addition, stirring is continued for about 6 hours until the iron complex is almost completely dissolved.

This solution is then filtered in a small press-plate filter for clarification.

The solution obtained is pre-heated to a temperature comprised between 40 and 60 degrees C and is sent on to a spray-drier, which uses filtered and heated air at a temperature of about 170-200 degrees C to dry the solution which has been atomised into small droplets. Thus the desired product is obtained in the form of a sodium salt having a granulometry which enables it to be easily dissolved in water for the preparation of the final liquid pharmaceutical forms.

### Step e')

The clarified solution according to the process described in step e) can be sent on for freeze-drying instead of spray-drying.

The product obtained with high qualitative characteristics and with a very low insoluble residue of <0.3% is however more expensive to produce than the product obtained using spray-drying.

### Step f) Product characteristics.

- the iron succinyl casein complex
- appearance: brown granular powder with slight milky odour; tasteless
- humidity < 5%
- solubility: soluble in purified H₂O
- pH = 7.5-9.0
- free iron:< 50ppm
- total iron: 4.8-5.4%
- proteins: >72.5%
- succinyl acid total: 7-9.5%
- Free succinyl acid: < 1.5%
- Chlorides: <2.5%
- Methylparabens: <3% in weight
- Propylparabens: <1% in weight
- Bacterial content: <10³ UFC/g
- Mold and yeast content:<10² UFC/g
- Insoluble residue:<0.2-0.3%

Example no. 2. Production of iron protein acetyl aspartate complex.

### Step a) Preparation of the acetyl aspartate casein

In a stainless steel reactor with sufficient shaker and pH detector, preferably an anchor stirrer, 200 litres of de-ionized, microbiologically pure water added to by 20 Kg of casein suitable for food or pharmaceutical use. The mixture is shaken to obtain a homogeneous suspension. 2.5 litres of caustic soda at 15% w/v are added to this over a period of about 20-30 minutes up until a pH of about 8 is reached. To this solution are added 5.0 Kg of succinyl anhydride and about 15.8 litres of a 15% caustic soda solution, maintaining pH at between 7.5 and 9.

The solution obtained is left to rest for between 1 and 2 hours. The reaction is conducted at a temperature of between 20 and 25 degrees celsius.

### Step b) Precipitation of the acetyl aspartate casein

The solution obtained in step a) is transferred into a glaze reactor, equipped with a high-speed stirrer to keep the solution homogeneous. This solution is slowly added to with 17-18 litres of HCl at 15% w/v up until a pH of about 3 is obtained. On reaching the correct pH addition of acid is stopped and a precipitate without salt and water-soluble impurities is obtained.

### Step c) Filtration and granulation.

The acetyl aspartate casein obtained in step b) is collected by filtration and washed with distilled water.

The product gathered from the filter is generally conformed in compact blocks which dissolve with difficulty.

To facilitate dissolving damp granulation (e.g. in a Viani granulator) of the filtered product is recommended.

The granulate is placed in a stainless steel reactor in which 200 litres of distilled water have been placed, and the mixture is shaken until a homogeneous suspension is obtained, to which about 9 litres of caustic soda at 15% w/v are added up until a pH of 8-9 is reached. During the final stage the suspension is stirred for about 6 hours in order to obtain an almost-complete dissolving of the acetyl aspartate casein.

### The solution is sent on to filtration.

The filtered solution is loaded in a glaze reactor equipped with a fast stirrer and with a pH detector, wherein over a period of about 30-40 minutes a solution of 6.0 ferrous chloride in 60 litres of water is added, up until a pH of 3 is reached.

When the pH of 3 is reached, addition of ferrous chloride is stopped. An iron acetyl aspartate complex precipitate is obtained, which is slowly shaken for about 30 minutes, whereupon the complex is filtered to gather the product thus obtained.

The reactor can be washed with distilled water to recuperate any product remaining in the reactor.

### Step d) Purification of the iron acetyl aspartate complex.

The iron acetyl aspartate complex obtained in step c) subjected to damp granulation is then loaded into a steel reactor where 300 litres of distilled water have been loaded.

The mixture is shaken for about 30 minutes to dissolve the salts and other water-soluble impurities and is then filtered.

### Step e) Clarification and spray-drying.

The washed and filtered and possibly granulated product is loaded into a steel anchor stirrer reactor into which 300 litres of distilled water have been loaded, and shaken for about an hour, whereupon 3.0 litres of a 15% caustic soda solution are added up until a pH of about 6 is reached.

The preservatives are added to this suspension, for example 1.34 kg of methylparaben in 5.0 litres of water and 0.36 kg of propylparaben in 5.0 litres of water.

About another 1 litre of 15% caustic soda solution is added to this suspension up until a pH of between 8 and 9 is reached.

During the addition, stirring is continued for about 6 hours until the iron complex is almost completely dissolved.

This solution is then filtered in a small press plate filter for clarification.

The solution obtained is pre-heated to a temperature comprised between 40 and 60 degrees C and is sent on to a spray-drier, which uses filtered and heated air at a temperature of about 170-200 degrees C to dry the solution which has been atomised into small droplets. Thus the desired product is obtained in the form of a sodium salt having a granulometry which enables it to be easily dissolved in water for the preparation of the final liquid pharmaceutical forms.

### Step e')

The clarified solution according to the process described in step e) can be sent on for freeze-drying instead of spray-drying.

The product obtained with high qualitative characteristics and with a very low insoluble residue of <0.3% is however more expensive to produce than the product obtained using spray-drying.

### Step f) Product characteristics.

- iron acetyl aspartate complex
- appearance: brown granular powder with slight milky odour; tasteless
- humidity < 5%
- solubility: soluble in purified H₂O
- pH = 7.5-9.0
- free iron:< 50ppm
- total iron: 4.8-5.4%
- proteins: >72.5%
- n-acetyl aspartic acid total: 7-9.5%
- Free n- acetyl aspartic acid: < 1.8%
- Aspartic acid <0.2%
- Chlorides: <2.5%
- Methylparabens: <3% in weight
- Propylparabens: <1% in weight
- Bacterial content: < 10³ UFC/g
- Mold and yeast content:<10² UFC/g
- Insoluble residue:<0.2-0.3%

## Claims

1. A process for producing an iron complex (III) with succinyl casein, obtained by reaction of casein with succinyl anhydride and a subsequent reaction of the succinyl casein obtained with ferrous chloride, wherein granulation of the succinyl anhydride and the iron succinyl casein is performed to facilitate dissolving thereof; the sodium salt iron succinyl casein is dried using a spray-drying or freeze-drying technique.

2. The process of claim 1, wherein addition of succinyl anhydride to the casein solution is performed with a pH of between 6 and 9.

3. The process of claim 1, wherein the succinyl casein obtained is precipitated by acidification at a pH comprised between 1 and 4.

4. The process of claim 1, wherein the reaction between the succinyl casein and the ferrous chloride solution is performed until a pH of 2.5-3 is obtained.

5. The process of claim 1, wherein the iron succinyl casein solution is clarified before being sent on for spray-drying or freeze-drying.

6. A process for producing an iron complex (III) with acetyl aspartic casein, obtained by reaction of casein with acetyl aspartic anhydride and following reaction of the acetyl aspartic casein obtained with ferrous chloride, wherein granulation of the acetyl aspartic casein and the iron acetyl aspartic casein is performed to facilitate dissolving thereof; the sodium salt iron succinyl casein is dried using a spray-drying or freeze-drying technique.

7. The process of claim 6, wherein addition of acetyl aspartic anhydride to the casein solution is performed with a pH of between 6 and 9.

8. The process of claim 6, wherein the acetyl aspartic casein obtained is precipitated by acidification at a pH comprised between 1 and 4.

9. The process of claim 6, wherein the reaction between the acetyl aspartic casein and the ferrous chloride solution is performed until a pH of 2.5-3 is obtained.

10. The process of claim 6, wherein the iron acetyl aspartic casein solution is clarified before being sent on for spray-drying or freeze-drying.

## Patentansprüche

1. Verfahren zur Herstellung eines Eisen(III)-Komplexes mit Succinylkasein, der durch Umsetzung von Kasein mit Bernsteinsaure anhydrid und folgende Reaktion des erhaltenen Succinylkaseins mit Eisenchlorid erhalten wird, worin die Granulation des Bernsteinsaure anhydrids und des Eisen-Succinylkaseins durchgeführt wird, um deren Auflösung zu vereinfachen; der Natriumsalz von Eisen-Succinylkasein wird durch eine Sprühtrocknungs- oder eine Gefriertrocknungstechnik getrocknet.

2. Verfahren nach Anspruch 1, worin die Zugabe von Bernsteinsaure anhydrid zur Kaseinlösung bei einem pH-Wert von 6 bis 9 durchgeführt wird.

3. Verfahren nach Anspruch 1, worin das erhaltene Succinylkasein wird durch Säuerung bei einem pH-Wert von 1 bis 4 ausgefällt.

4. Verfahren nach Anspruch 1, worin die Reaktion von Succinylkasein mit der Eisenchloridlösung durchgeführt wird, bis ein pH-Wert von 2,5-3 erhalten wird.

5. Verfahren nach Anspruch 1, worin die Eisen-Succinylkasein-Lösung vor der Sprühtrocknung oder Gefriertrocknung geklärt wird.

6. Verfahren zur Herstellung eines Eisen(III)-Komplexes mit Acetylasparaginkasein, der durch Umsetzung von Kasein mit Acetylasparaginanhydrid und folgende Umsetzung des erhaltenen Acetylasparaginkaseins mit Eisenchlorid erhalten wird, worin die Granulation des Acetylasparaginkaseins und des Eisen-Acetylasparaginkaseins durchgeführt wird, um deren Auflösung zu vereinfachen; der Natriumsalz von Eisen-Succinylkasein wird durch eine Sprühtrocknungs- oder eine Gefriertrocknungstechnik getrocknet.

7. Verfahren nach Anspruch 6, worin die Zugabe von Acetylasparaginanydrid zur Kaseinlösung bei einem pH-Wert von 6 bis 9 durchgeführt wird.

8. Verfahren nach Anspruch 6, worin das erhaltene Acetylasparaginkasein wird durch Säuerung bei einem pH-Wert von 1 bis 4 ausgefällt.

9. Verfahren nach Anspruch 6, worin die Umsetzung von Acetylasparaginkasein mit der Eisenchloridlösung durchgeführt wird, bis ein pH-Wert von 2,5-3 erhalten wird.

10. Verfahren nach Anspruch 6, worin die Eisen-Acetylasparaginkasein-Lösung vor der Sprühtrocknung oder Gefriertrocknung gereinigt wird.

## Revendications

1. Procédé de production d'un complexe de fer (III) avec succinylcaséine, obtenu par réaction de caséine avec anhydride succinique et ensuite réaction de la succinylcaséine obtenue avec chlorure ferreux, où la granulation de la l'anhydride succinique et de la ferro-succinylcaséine est réalisée pour en simplifier la dissolution; le sel de sodium de ferro-succinylcaséine est séché par une technique de séchage par pulvérisation ou de lyophilisation.

2. Procédé selon la revendication 1, où l'addition de succinylanhydride à la solution de caséine est réalisée avec un pH de 6 à 9.

3. Procédé selon la revendication 1, où la succinylcaséine obtenue est précipitée par acidification à un pH de 1 à 4.

4. Procédé selon la revendication 1, où la réaction de la succinylcaséine avec la solution de chlorure ferreux est réalisée jusqu'à obtenir un pH de 2,5-3.

5. Procédé selon la revendication 1, où la solution de ferro-succinylcaséine est clarifiée avant le séchage par pulvérisation ou lyophilisation.

6. Procédé de production d'un complexe de fer (III) avec caséine acétyl-aspartique, obtenu par réaction de caséine avec anhydride acétyl-aspartique et ensuite réaction de la caséine acétyl-aspartique obtenue avec chlorure ferreux, où la granulation de la caséine acétyl-aspartique et de la ferro-caséine acétyl-aspartique est réalisée pour en simplifier la dissolution; le sel de sodium de ferro-succinylcaséine est séché par une technique de séchage par pulvérisation ou lyophilisation.

7. Procédé selon la revendication 6, où l'addition de anhydride acétyl-aspartique à la solution de caséine est réalisée avec un pH de 6 à 9.

8. Procédé selon la revendication 6, où la caséine acétyl-aspartique obtenue est précipitée par acidification à un pH de 1 à 4.

9. Procédé selon la revendication 6, où la réaction de la caséine acétyl-aspartique avec la solution de chlorure ferreux est réalisée jusqu'à obtenir un pH de 2,5-3.

10. Procédé selon la revendication 6, où la solution de ferro-caséine acétyl-aspartique est clarifiée avant le séchage par pulvérisation ou lyophilisation.
